Europäisches Patentamt

(19) European Patent Office

Office européen des brevets

(11) Publication number: **0 081 881**
**B1**

(12) **EUROPEAN PATENT SPECIFICATION**

(45) Date of publication of patent specification: **01.04.87**

(21) Application number: **82201554.1**

(22) Date of filing: **07.12.82**

(51) Int. Cl.⁴: **C 11 B 7/00,** A 23 D 5/00,
C 11 C 3/00, A 23 L 1/24,
A 61 K 7/00, C 10 M 105/32,
C 10 L 1/18, A 61 K 31/20,
A 61 K 31/23

(54) **A process for the solvent fractionation of palm oil stearines and products obtained with said process.**

(30) Priority: **15.12.81 IT 2561581**

(43) Date of publication of application:
**22.06.83 Bulletin 83/25**

(45) Publication of the grant of the patent:
**01.04.87 Bulletin 87/14**

(84) Designated Contracting States:
**AT BE CH DE FR GB IT LI NL SE**

(56) References cited:
**EP-A-0 035 298**
**FR-A-2 415 138**

(73) Proprietor: **SOCIETE DES PRODUITS NESTLE S.A.**
**Case postale 353**
**CH-1800 Vevey (CH)**

(72) Inventor: **Luddy, Francis E.**
**611 Orchard Way**
**Hatboro-PA 19040 (US)**
Inventor: **Longhi, Sergio**
**Via Verdi, 25**
**I-43100 Parma (IT)**

Courier Press, Leamington Spa, England.

## Description

The present invention relates to a process for the solvent fractionation of palm oil stearines and to the products obtained with said process.

Palm oil is a semisolid fat obtained from the oil palm *Elaeis guineensis* which is cultivated mainly in Malaysia, West and Central Africa, Brazil and other South American countries. The world wide production in recent years has reached over 7 billion pounds (about $3 \times 10^9$ Kg). Palm oil is a semisolid product whose triglycerides contain almost equal amounts of saturated and unsaturated fatty acids. Because of its solid nature, it has some limitations as a food fat and today various processes are employed to modify the physical properties of palm oil. Large quantities of palm oil are fractionated by either thermal winterization or by detergent fractionation into stearine and oil fractions. The fractionations are not completely selective but they do produce oil fractions as the major component. The oils despite limitations in their physical properties find a market as economical replacements for the more expensive salad and cooking oils. Such is not the case for the stearine portion of the fractionation products. Palm oil stearine finds very limited use as a food fat. The major component of the stearine fraction is the residual oil and the mixture is too soft to replace high melting fats in shortenings and margarines or for other typical uses of hard fat. It is a surplus fat of low economic value and few uses.

The confectionery industry annually uses many millions of pounds of cocoa butter in the manufacture of chocolates and other confections. Cocoa butter is desirable as a confectionery fat because of its unique physical properties. It is a solid fat up to about 24°C, after which it begins to soften and it melts sharply at about 35—37°C. Because of this prized melting property, cocoa butter contributes greatly to "flavor release" and "mouth feel" of candy and confections. However, the supply of cocoa butter is limited since the cocoa bean plants are grown in a very narrow climate zone and their cultivation is difficult. For this reason many attempts have been made to produce cocoa butter-like fats from more abundant and economical fat sources. This is one of the objectives of the present invention, particularly, since none of the known methods of fat fractionation described in U.S. 3,541,123, 3,686,240, 2,975,062, 3,431,116, 4,127,597 and 3,944,585, FR 2 415 138, GB 1,006,041 and EP—A—0035298 can satisfactorily carry out the processes of this invention.

Specifically, FR 2 415 138 describes the fractionation of palm oil or of butyrin, but gives no suggestion how palm oil stearine (normal or rearranged) might be handled with respect to crystallization temperatures. Likewise, EP—A—0035298 is concerned exclusively with olive oil stearine and again contains no indication how, or whether, the process described might be adapted to the fractionation of palm oil stearines.

The novelty and advantages of this invention over those previously cited depend to a great extent on several important factors.

(1) The process has been designed expressly for the fractionation of palm oil stearine and catalytically rearranged palm oil stearine.

(2) The inventors in this process pay particular attention to the size and nature of the precipitated crystals by emphasizing the need for slowly approaching the crystallization temperature. This technique produces large, filterable crystals of the more desirable polymorphic forms.

(3) The invention recognizes that agitation can gainfully accelerate the equilibrium process but also recognizes that indiscriminate agitation can fragment existing crystals making isolation of the precipitates inefficient or impossible. Also, too rapid agitation leads to excessive supercooling which promotes formation of less stable crystal forms.

(4) Previously, inventors have shown little concern with treatment of the crystalline fractions after separation from the crystallization medium. This invention stresses that the separation of desirable from non-desirable components in the isolated crystals is very important in producing fractions of specific properties and narrow melting range. To this extent, the inventors have added a special "extraction step" which efficiently in one operation removes all occluded lower melting glyceride components from the isolated precipitates.

Prior inventors have described the products of their inventions by classical analytical values such as iodine numbers, saponification values, capillary melting points and solid fat index. These classical values give little specific information about the composition or the performance of the triglyceride products. We prefer to characterize the products of this invention by the technique of Differential Scanning Calorimetry in which melting behavior of the products as they change from the solid to the liquid state over a temperature range is depicted graphically as a melting profile. Likewise, instead of enumerating the general unsaturation or the average molecular weights of these materials, we prefer to obtain the actual fatty acid composition by gas liquid chromatography which describes the molecular weight and the unsaturation of the component fatty acids.

Accordingly, in this invention we shall disclose the process for fractionation of palm oil stearine or for catalytically rearranged palm oil stearine by a combination of solvent and temperature to provide easily separable crystals readily collected by decantation, filtration or centrifugation. Catalytic rearrangement of palm oil stearine makes little obvious alteration in the nature of the material and the fractionation products of each are similar in yield and in melting behavior. The fatty acid composition of a typical palm oil stearine is compared with that of rearranged palm oil stearine in Table A:

2

**0 081 881**

TABLE A
Fatty acid composition-wt.%
Palm oil stearines

| Acid | normal | rearranged |
|---|---|---|
| palmitic | 54 | 48 |
| stearic | 5 | 7 |
| oleic | 33 | 34 |
| linoleic | 7 | 7 |
| others | 1 | 4 |

The graphical representation of the fractionation process is shown in Table B which also gives the solvent to fat ratios and the temperatures of crystallization:

TABLE B
Fractionation steps—Palm oil stearines

1000 grams—Palm oil stearines

stirring rate 35—70 RPM
crystallization time—2 hours

solvent—acetone
solvent ratio—4.5 to 1
crystallization temp. 12—14°C

Initial crystall.

Precipitate → Extraction

Filtrate—F-1

stirring-35—70 RPM, 2 hours

cryst. temp. −1 to −3°C
solvent ratio—same

Hard fat fraction, P-1

Initial crystall.

Precipitate → Extraction

Filtrate → Oil fraction F-2

Confectionery fat fraction P-2

The yield of fractionation products for both types of palm oil stearines is given in Table C:

TABLE C
Fractionation of palm oil stearines
percent yield

| Fraction | normal | rearranged |
|---|---|---|
| Hard fat, P-1 | 25 | 23 |
| Confectionery fat, P-2 | 27 | 24 |
| Oil, F-2 | 48 | 53 |

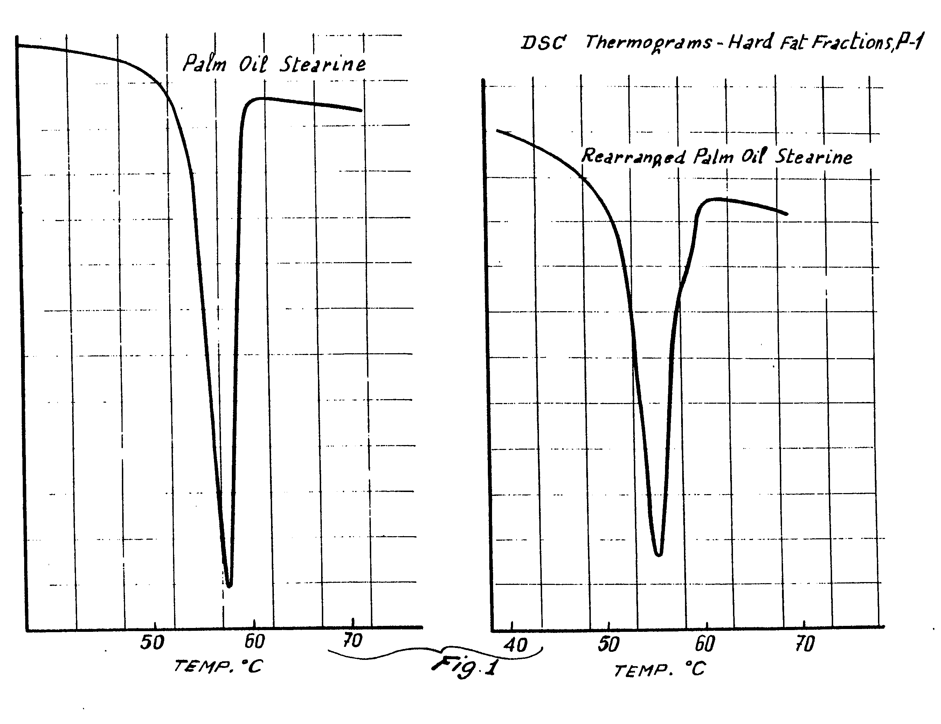

The first fraction collected is a hard, solid fat, Fraction P-1, which melts above 45°C. The DSC melting profiles of these fractions are shown in Figure 1 and the fatty acid compositions of these fractions are given in Table D:

3

TABLE D
Fatty acid composition—wt.%
Palm oil stearines—Hard fat fraction, P-1

| Acid | normal | rearranged |
|------|--------|------------|
| palmitic | 83 | 76 |
| stearic | 7 | 10 |
| oleic | 7 | 9 |
| linoleic | 1 | 1 |
| others | 2 | 4 |

Both fractions melt sharply with peak melting of 56°C for the rearranged and a little higher for the normal stearine. These hard fractions can be used to harden shortenings and margarines or blended with the oil fraction or other oil to make speciality shortenings. The high concentration of palmitic acid in the fractions makes them valuable for many non-food uses including cosmetics and pharmaceuticals and as a commercial source of palmitic acid. The second crystalline fraction of the process, Fraction P-2, is also a solid fat fraction but of lower melting range than the first fraction, P-1. The melting range of this fraction is usually from 33—44°C and it is known as the confectionery fat fraction. Its melting properties are very similar to those of cocoa butter and it has many useful applications in the bakery and confectionery industries. Since it melts near body temperature, it also finds use in the cosmetic industry as a cream and lotion base and in the pharmaceutical industry as a drug carrier. The fatty acid compositions of the confectionery fat fractions from both normal and randomized palm oil stearines are given in Table E:

TABLE E
Fatty acid composition—wt.%
Palm oil stearines—Confectionery fat fr., P-2

| Acid | normal | rearranged |
|------|--------|------------|
| palmitic | 61 | 57 |
| stearic | 6 | 9 |
| oleic | 27 | 28 |
| linoleic | 4 | 4 |
| others | 2 | 2 |

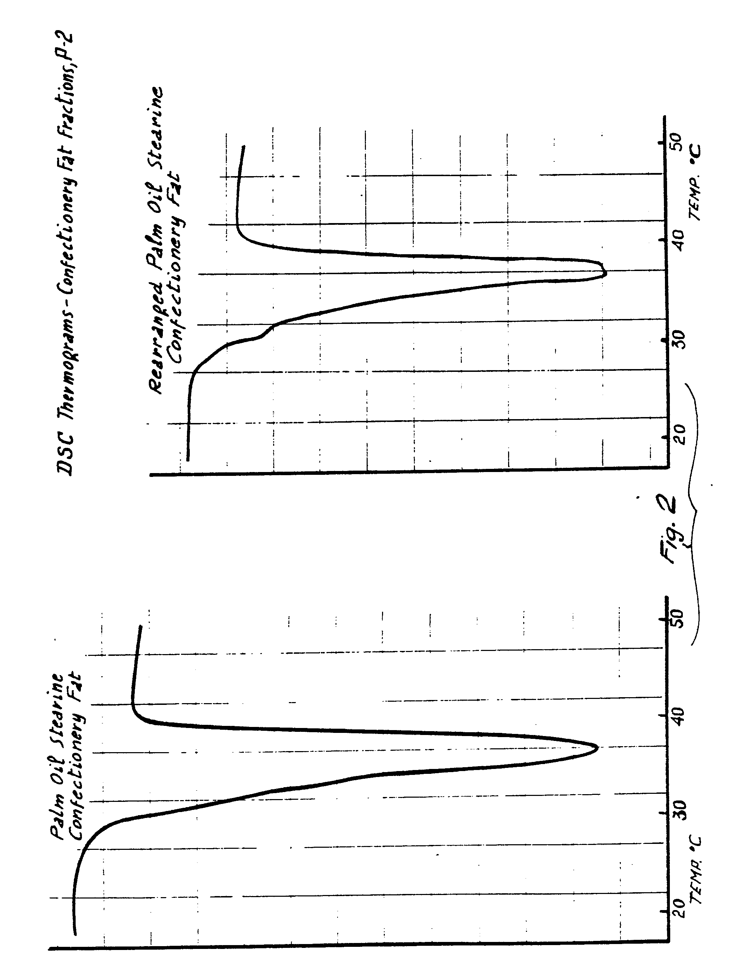

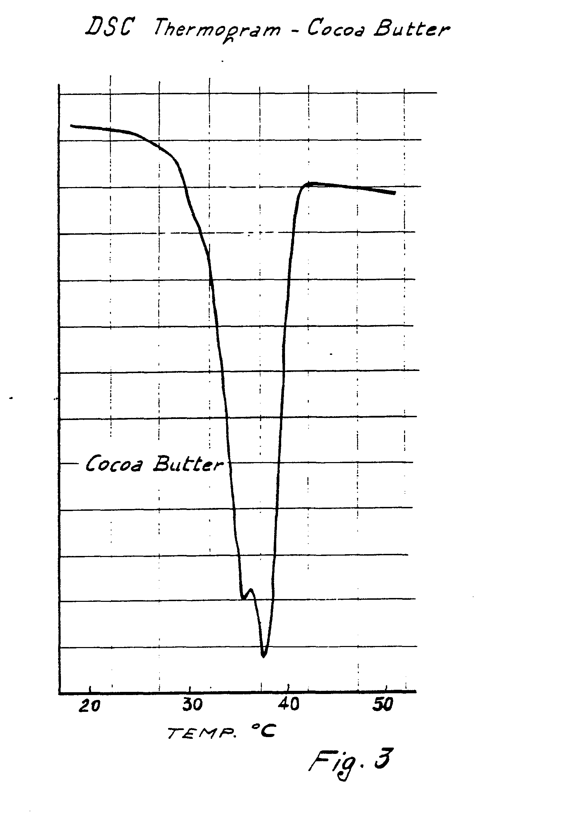

The unique thermal properties of the fractions are demonstrated by the DSC thermal profile of each shown in Figure 2. Both fractions exhibit peak melting at 37°C. For comparison, the melting profile for cocoa butter is shown in Figure 3 and it also exhibits sharp melting at 37°C. The resemblence in the melting properties of the stearines' confectionery fat fractions and cocoa butter is evident. The third fraction of this process, Fraction F-2, is an oil at room temperature. The oil fraction is the major fraction of both types of palm oil stearine. As an oil, the fraction is particularly useful. It is superior to the oil fraction obtained from palm oil by conventional thermal or detergent fractionation since it has a lower solidification temperature. It has many food uses as a salad, cooking or frying oil and it can be blended with the hard fat fraction, P-1, to produce margarines or speciality shortenings. In the non-food category, it can be used for making lotions or ointments in cosmetics or pharmaceuticals. It can also be used as a commercial source of oleic acid, as an extender for diesel fuels, as a base for synthetic lubricants or for the manufacture of foam plastics. Although it has the same properties as many seed oils, it is much more stable chemically. Thus, it could be used more effectively as a diesel fuel extender in place of seed oils now being investigated (Chemistry and Engineering News 59, #36, 69—77, (1981)). Since the palm oil stearine is a low cost, surplus fat, the economics of the process may be very favorable in view of the high cost of hydrocarbon fuels. It also has the advantage of being a renewable natural resource. The fatty acid compositions of the oil fractions from palm oil stearine and rearranged palm oil stearine are given in Table F:

TABLE F
Fatty acid composition—wt.%
Palm oil stearines—Oil fraction, F-2

| Acid | normal | rearranged |
|------|--------|------------|
| palmitic | 39 | 42 |
| stearic | 3 | 5 |
| oleic | 44 | 41 |
| linoleic | 11 | 11 |
| others | 3 | 1 |

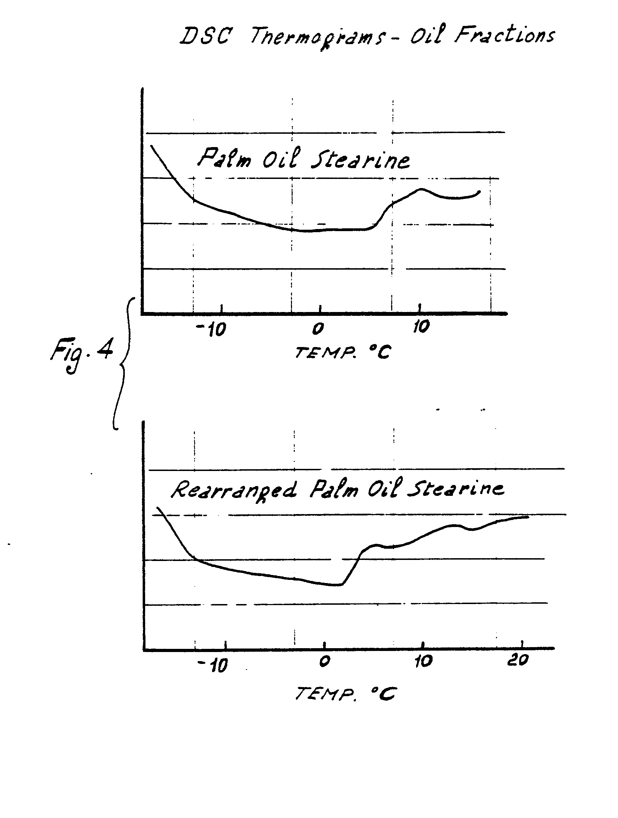

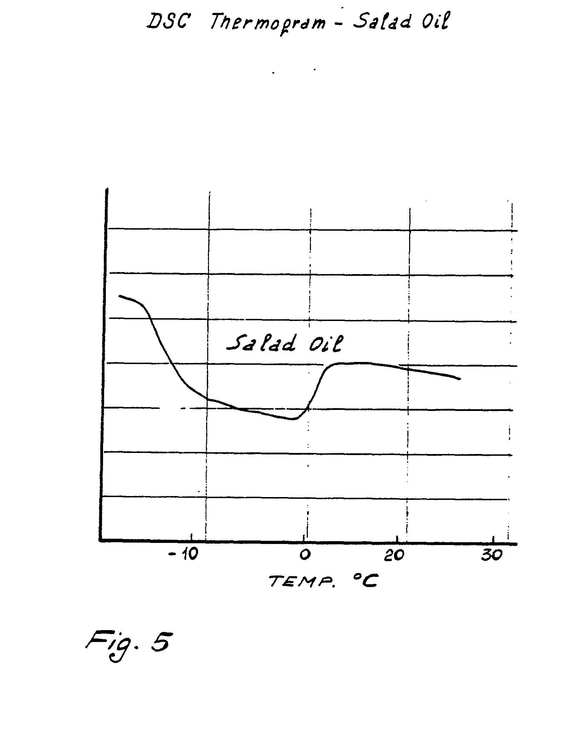

The thermal properties of the oil fractions are shown by the DSC thermogram of Figure 4. For comparison, the DSC thermogram of commercial salad oil is shown in Figure 5.

The processes of this invention outlined graphically in Table B preferably comprise the following individual steps:

(a) dissolving the melted stearine in the appropriate solvent using a solvent to fat ratio of 1.0 to 6.0 ml of solvent per gram of fat, warming the mixture to about 50°C to ensure complete solution,

(b) crystallizing the mixture of Step a at 12—14°C,

(c) collecting the precipitate formed in Step b by vacuum or pressure filtration or by centrifugation while maintaining the work area at low humidity and a temperature of 15—20°C,

(d) transferring the collected precipitate of Step c to a suitable vessel, adding about twice the precipitate volume of fresh solvent, usually less than 10% of the original crystallization volume, cooled to about 2°C below the crystallization temperature and stirring the mixture gently to produce a slurry of solvent and precipitate,

(e) filtering the slurry of Step d under vacuum or pressure, or by centrifugation, and collecting the precipitate,

(f) processing the collected precipitate of Step e in the evaporator and the deodorizer to produce the bland, solvent-free first fraction, P-1,

(g) further crystallizing the filtrate from Step c at a temperature of 1—3°C,

(h) collecting the precipitate from Step g by vacuum or pressure filtration or by centrifugation while maintaining the work area at low humidity and a temperature of 10—15°C,

(i) transferring the collected precipitate of Step h to a suitable vessel, adding about twice its volume of fresh solvent previously cooled to 0°C, slowly stirring the mixture to produce a slurry of solvent and precipitate,

(j) filtering the slurry of Step i by vacuum or pressure filtration or by centrifugation and collecting the precipitate,

(k) processing the collected precipitate of Step j in the evaporator and deodorizor to produce the bland, solvent-free second fraction, P-2, the confectionery fat fraction,

(l) processing the collected filtrate of Step h in the evaporator and the deodorizer to produce the bland, solvent-free, third fraction, F-2, the oil fraction,

(m) collecting the filtrates from Step e and from Step j, the extraction steps, for use directly as solvent for the initial crystallization Step, thus minimizing solvent recovery.

Although the process of this invention may be carried out by either the batch or continuous process, the crystallizer design must balance the size and growth of the crystals with capacity. In many cases, the batch process requiring very simple equipment and readily controlled parameters of crystallization may be the process of choice. In any case, we advocate controlled crystallizations in either the batch or continuous processes using scraped surface crystallizers.

We are in agreement with A. E. Bailey, Industrial Oil and Fat Products, 3rd edition, Chapt. 21, edited by Daniel Swern, Interscience Publishers, N.Y., N.Y., (1964), "It is essential that the crystals be formed slowly so that they will be large and easily separated". Likewise, G. Singh, American Institute of Chemical Engineers, Symposium Series, #153, Vol. 72, page 100—109 (1976), "Therefore controlled crystallization to maximize the filterability of the crystal is the key to the successful operation of the processes". We have found the stirring rate of 35—70 RPM to be ideal in the batch process for inducing the formation of crystals most suitable for filtration. In experiments using continuous scraped surface crystallizers, modest dwell times of 5 to 50 minutes are desired to produce crystals large enough to be readily filterable. We prefer the use of tandem and parallel crystallizers to increase throughput.

We also advocate the additional "extraction step" to the isolation of the crystalline precipitates of the process. In any filtration step the precipitate is always accompanied by varying amounts of occluded filtrate solution. These occluded lower melting components could seriously affect the melting properties and chemical stability of the crystalline material. Contamination of this kind in a confectionery fat fraction

5

would render the fraction unsuitable for confectionery which requires a specific narrow melting range. Prior inventors have depended on washing the crystals on the filter to remove the lower melting contaminants. The washing step by its very nature is inefficient and requires large volumes of fresh solvent. Accordingly, in this invention, it has been discovered that the crystals isolated by filtration or centrifugation are extremely stable and show no tendency to melt if the work area is maintained between 10—20°C. Thus, the crystals can be removed from the filter and transferred to a suitable vessel to carry out not a washing but an "extraction" step. The extraction step efficiently removes the lower melting components from the crystals with little or no loss of the desired product. For the fractionation of palm oil stearine, only one extraction step was required but two or more could be carried out without loss of crystal material. The added extraction step has several advantages;

(a) It is not a crystallization step, hence it does not require heating and melting of the precipitate followed by large additions of solvent to solubilize the fat and recooling under controlled conditions to the selected crystallization temperature.

(b) The extraction step removes the lower melting components yielding a purer high melting fraction. This is important in producing the desired thermal characteristics in the confectionery fat fraction.

(c) The filtrate from the extraction step can be used directly as solvent for the initial crystallization step minimizing solvent recovery.

The process of the invention may be carried out either as a batch or continuous process but for the purpose of the disclosure only the batch process will be exemplified in detail. The solvent to be used is an extremely important factor in the solvent partitioning of fats and for our purpose acetone is the preferred solvent. In our process solvents such as methyl and ethyl alcohol, isopropyl alcohol, methyl ethyl ketone, mixed hydrocarbons with boiling points under 130°C, 1-nitropropane, 2-nitropropane, and various mixtures of these solvents may also be used.

The fractions obtained, P-1, P-2 and F-2 may be used alone or each can be blended with other materials to provide products adopted for specific purposes.

Examples of these are:

— a confectionery fat comprising 1 to 99% by weight of P-2 and 99 to 1% by weight of cocoa butter;
— a confectionary fat having a higher melting point than cocoa butter comprising 5 to 20% by weight of P-1 and 95 to 80% by weight of P-2;
— a salad, cooking or frying oil comprising 1 to 99% by weight of F-2 and 99 to 1% by weight of an oil such as olive, peanut, soybean, corn, palm, sunflower seed, safflower or rapeseed oil.


Example
Fractionation of palm oil stearines according to Table B.

A mixture of 1000 grams of melted palm oil stearine or catalytically rearranged palm oil stearine and 4500 ml of acetone (solvent ratio—4.5 ml of acetone per gram of stearine) was heated to about 50°C until all of the stearine was in solution. The solution was transferred to a crystallizer equipped with mechanical stirring and cooling coils. The solution was slowly stirred, first at 75 RPM and then gradually lowered until a rate of 35 RPM was reached. The cooling medium in the cooling coils was maintained at no more than 5°C below the temperature of the crystallizer, except for the final crystallization temperatures, when the differential was only 2°C. The solution temperature was lowered gradually over a period of one hour to about 15°C and stirring was stopped at 12°C. Crystallization began at about 28°C and became quite heavy at about 22°C. When the temperature of 12°C was reached, the mixture was held for an additional 15 min. at this temperature without stirring. During this period the fat crystals settled rapidly to the bottom of the crystallizer. Most of the clear supernatant liquid was removed by syphoning using a slight vacuum and was transferred to the crystallizer holding tank prior to the next crystallization. The remaining precipitate and filtrate solution was filtered using slight vacuum and the precipitate and filtrate were collected. The filtrate was added to the previously syphoned filtrate and the combined filtrates (F-1) were transferred to the crystallizer for the next crystallization step. The precipitate filter cake, almost crystalline in appearance, was transferred to a wash vessel and about 600 ml of fresh acetone previously cooled to 10°C was added and the mixture gently stirred until a slurry of precipitate and solvent was formed. The slurry was immediately filtered using slight vacuum. The extracted, filtered precipitate was treated first in the evaporator to remove residual solvent and then deodorized. The final yield of this bland, high melting fat (mp 56°C), was 270 grams. This is the P-1 fraction of the scheme outlined in Table B, and its DSC thermal profile is shown in Figure 1. The filtrate from the extraction step contained about 10 grams of fat or about 4% of the hard fat fraction (P-1). If not removed, it would have contaminated the hard fat fraction with lower melting materials. The collected filtrate (P-1 extract) can be used in place of fresh solvent in the first crystallization step of a new crystallization cycle.

In the second crystallization step, the temperature of the filtrate solution (F-1), from the first crystallization was about 15°C after being transferred to the crystallizer. The temperature of the cooling medium was adjusted to 10°C and the differential temperature of 5°C between cooling medium and sample was gradually decreased over the next hour until the temperature of the cooling medium was 0°C and the sample temperature in the crystallizer was 2°C. In the same time period, the stirring rate initially started at 75 RPM was also gradually lowered to 35 RPM when the crystallization temperature was 8°C. Stirring was stopped when the sample temperature reached 2°C and the crystallization mixture was then held for 15 minutes without stirring. In this step, crystals appeared at 11°C and the precipitate was quite heavy at 8°C. In the non-stirring period, all of the precipitate crystals settled rapidly to the bottom of the crystallizer and most of the filtrate volume was removed by syphoning and transferred to a holding tank. The remaining mixture of precipitate and filtrate was filtered using mild vacuum. The combined filtrates from the syphoning and filtration steps represent F-2, of the fractionation scheme shown in Table B, and after processing in the evaporator and the deodorizer yielded 450 grams of a bright, light yellow, bland oil. The thermal characteristics of this fraction are shown in the thermogram of Figure 4 and for comparison the DSC thermogram of soybean oil is shown in Figure 5. The precipitate filter cake, although not as dry and crystalline as the P-1 filter cake was readily transferred to the extraction tank. During this time, the work area was maintained at about 10°C with low humidity and there was no visible sign of melting in the precipitate crystals. 600 ml of acetone, cooled to about 0°C, were added and the mixture gently stirred until a slurry of precipitate and solvent was formed. The slurry was immediately filtered using slight vacuum. The collected precipitate was processed in the evaporator to remove solvent and then deodorized under standard conditions. The yield of bland, confectionery fat material (P-2 of the fractionation scheme Table B) was 280 grams. The thermal melting profile of this material is shown in Figure 2. The DSC thermal profile of cocoa butter is shown in Figure 3 and a comparison of the two thermograms indicates that they have similar melting characteristics. The filtrate solution from the extraction step, P-2 extract, can be combined with the solvent from the first extraction step, P-1 extract, and can be used in place of fresh solvent in the first crystallization step of a new fractionation cycle. The P-2 extract contained about 20 grams of material equal to about 7% of the confectionery fat fraction. If not removed it would have contaminated the confectionery fat fraction, P-2, with low melting oil components. The adjusted yield of each fraction, also given in Table B, is hard fat fraction, P-1, 270 grams or 27%, confectionery fat fraction, P-2, 280 grams or 28% and the oil fraction, F-2, 450 grams or 45%.

## Claims

1. A process for the solvent fractionation of palm oil stearine or catalytically rearranged palm oil stearine yielding as the first fraction, a hard glyceride fat, with melting point above 45°C, as second fraction a semisolid glyceride fraction resembling cocoa butter in melting properties and having a melting range in excess of 33°C and under 44°C and as third fraction an oil liquid at room temperature, the process being characterized in that it comprises the steps of:

(a) dissolving the stearine in a solvent, the solvent ratio being from 1.0 to 6.0 ml of solvent per gram of stearine, warming the mixture to ca 50°C to ensure complete solution;

(b) crystallizing the solution from step (a) at 12—14°C;

(c) collecting the precipitate formed in step (b) by filtration or centrifugation;

(d) transferring the collected precipitate of step (c) to a suitable vessel and extracting the said precipitate with fresh solvent cooled to 2°C below the crystallization temperature and equal in volume to about twice the volume of the precipitate;

(e) filtering the slurry of step (d) by vacuum or pressure filtration or by centrifugation and collecting the precipitate, which after processing in the evaporator and deodorizer yields the bland, solvent free first fraction, (P-1), the hard fat fraction with melting point above 45°C;

(f) further crystallizing the filtrate from step (c) at a temperature of 1—3°C;

(g) collecting the precipitate formed in step (f) by filtration or centrifugation;

(h) transferring the collected precipitate of step (g) to a suitable vessel and extracting the said precipitate with fresh solvent cooled to 0°C and equal in volume to twice the volume of the precipitate;

(i) filtering the slurry of step (h) by vacuum or pressure filtration or by centrifugation and collecting the precipitate, which after processing in the evaporator and deodorizer, yields the bland, solvent free second fraction, (P-2), the confectionery fat fraction, of melting point over 33°C and under 44°C;

(j) processing the collected filtrate of step (g) in the evaporator and the deodorizer to produce the bland, solvent free third fraction, (F-2), the oil fraction.

2. A process according to Claim 1, wherein the solvent is acetone.

3. A process according to Claim 1, wherein the solvent is methyl ethyl ketone, methyl isobutyl ketone, methyl, ethyl or isopropyl alcohol, 1 or 2-nitropropane or a mixed hydrocarbon with boiling point under 130°C.

4. A batch process according to Claim 1, 2 or 3 wherein moderate stirring of 35—70 RPM is provided to accelerate the crystallization steps.

5. A continuous process according to Claim 1, 2 or 3 wherein modest dwell times of 5—50 minutes are provided in scraped surface crystallizers for accelerating the crystallization steps and forming the right sized crystal polymorphs.

6. A confectionery fat comprising from 1% to 99% by weight of fraction P-2 obtained by the process of any preceding claim and from 1% to 99% by weight of cocoa butter.

7. A confectionery fat of higher melting point than cocoa butter comprising a blend of 5% to 20% by weight of fraction P-1 and from 80%—95% by weight of fraction P-2, fractions P-1 and P-2 being prepared by a process according to any one of Claims 1 to 5.

7

**0 081 881**

8. A salad, cooking or frying oil comprising from 1% to 99% by weight of fraction F-2 obtained by a process according to any one of Claims 1 to 5 and from 1% to 99% by weight of olive oil, peanut oil, soybean oil, corn oil, palm oil, sunflower seed oil, safflower oil or rapeseed oil.

9. A speciality shortening or margarine comprising a blend of fractions P-1 and F-2 obtained by a process according to any one of Claims 1 to 5.

10. A cosmetic or pharmaceutical product having as base material fraction P-1 obtained by a process according to any one of Claims 1 to 5.

11. A cosmetic or pharmaceutical product having as base fraction P-2 or F-2 obtained by a process according to any one of Claims 1 to 5.

12. A synthetic lubricant or diesel fuel extender having as a base fraction F-2 obtained by a process according to any one of Claims 1 to 5.

**Patentansprüche**

1. Verfahren zur Lösungsmittelfraktionierung von Palmölstearin oder katalytische umgelagertem Palmölstearin, das als erste Fraktion ein hartes Glyceridfett mit einem Schmelzpunkt über 45°C, als zweite Fraktion eine halbfeste Glyceridfraktion, die im Schmelzverhalten Kakaobutter ähnelt und einen Schmelzbereich von über 33°C bis unter 44°C aufweist, und als dritte Fraktion ein bei Raumtemperatur flüssiges Öl ergibt, welches Verfahren dadurch gekennzeichnet ist, daß es die folgenden Stufen umfaßt:

(a) Auflösen des Stearins in einem Lösungsmittel, wobei das Lösungsmittelverhältnis von 1,0 bis 6,0 ml Lösungsmittel je Gramm Stearin beträgt, und Erwärmen des Gemisches auf etwa 50°C, um eine vollständige Lösung zu erzielen;

(b) Kristallisieren der Lösung aus Stufe (a) bei 12 bis 14°C;

(c) Gewinnen des in Stufe (b) gebildeten Niederschlages durch Filtration oder Zentrifugation;

(d) Überführen des aus Stufe (c) gewonnenen Niederschlages in ein geeignetes Gefäß und Extrahieren dieses Niederschlages mit frischem, auf 2°C unterhalb der Kristallisationstemperatur abgekühltem Lösungsmittel, dessen Volumen etwa dem zweifachen Volumen des Niederschlages entspricht;

(e) Filtrieren der Aufschlämmung aus Stufe (d) durch Vakuum- oder Druckfiltration oder durch Zentrifugation und Gewinnen des Niederschlages, der nach Verarbeitung im Evaporator und Desodorisator die blande, lösungsmittelfreie erste Fraktion, (P-1), die Hartfettfraktion mit einem Schmelzpunkt über 45°C, ergibt;

(f) weiteres Kristallisieren des Filtrats aus Stufe (c) bei einer Temperatur von 1 bis 3°C;

(g) Gewinnen des in Stufe (f) gebildeten Niederschlages durch Filtration oder Zentrifugation;

(h) Überführen des aus Stufe (g) gewonnenen Niederschlages in ein geeignetes Gefäß und Extrahieren dieses Niederschlages mit frischem, auf 0°C gekühltem Lösungsmittel, dessen Volumen dem zweifachen Volumen des Niederschlages entspricht;

(i) Filtrieren der Aufschlämmung aus Stufe (h) durch Vakuum- oder Druckfiltration oder durch Zentrifugation und Gewinnen des Niederschlages der nach Verarbeitung im Evaporator und Desodorisator die blande, lösungsmittelfreie zweite Fraktion, (P-2), die Süßwarenfettfraktion, mit einem Schmelzpunkt von über 33°C und unter 44°C ergibt;

(j) Verarbeiten des aus Stufe (g) aufgefangenen Filtrats im Evaporator und Desodorisator zur Ausbildung der blanden, lösungsmittelfreien dritten Fraktion, (F-2), der Ölfraktion.

2. Verfahren nach Anspruch 1, worin das Lösungsmittel Aceton ist.

3. Verfahren nach Anspruch 1, worin das Lösungsmittel Methylethylketon, Methylisobutylketon, Methyl-, Ethyl- oder Isopropylalkohol, 1- oder 2-Nitropropan oder ein Kohlenwasserstoffgemisch mit einem Siedepunkt unter 130°C ist.

4. Ansatzweises Verfahren gemäß Anspruch 1, 2 oder 3, worin ein mäßiges Rühren bei 35 bis 70 Umdrehungen je Minute zur Beschleunigung der Kristallisationsstufen vorgesehen wird.

5. Kontinuierliches Verfahren gemäß Anspruch 1, 2 oder 3, worin mäßige Verweilzeiten von 5 bis 50 min in Kratzkristallisatoren zur Beschleunigung der Kristallisationsstufen und Ausbildung von polymorphen Kristallen mit der richtigen Größe eingehalten werden.

6. Fett für Süßwaren, umfassend 1 bis 99 Gew.-% Fraktion P-2, die nach dem Verfahren gemäß einem der vorstehenden Ansprüche erhalten ist, und 1 bis 99 Gew.-% Kakaobutter.

7. Fett für Süßwaren mit höherem Schmelzpunkt als Kakaobutter, umfassend ein Gemisch aus 5 bis 20 Gew.-% Fraktion P-1 und 80 bis 95 Gew.-% Fraktion P-2, welche Fraktionen P-1 und P-2 nach einem Verfahren gemäß einem der Ansprüche 1 bis 5 hergestellt sind.

8. Salat-, Koch- oder Bratöl, umfassend 1 bis 99 Gew.-% Fraktion F-2, erhalten nach einem Verfahren gemäß einem der Ansprüche 1 bis 5, und 1 bis 99 Gew.-% Olivenöl, Erdnußöl, Sojaöl, Maisöl, Palmöl, Sonnenblumenöl, Safloröl oder Rapsöl.

9. Spezialbackfett oder Margarine, umfassend ein Gemisch aus Fraktionen P-1 und F-2, erhalten nach einem Verfahren gemäß einem der Ansprüche 1 bis 5.

10. Kosmetisches oder pharmazeutisches Produkt, das als Basismaterial eine nach einem Verfahren gemäß einem der Ansprüche 1 bis 5 erhaltene Fraktion P-1 aufweist.

11. Kosmetisches oder pharmazeutisches Produkt, das als Basis eine nach einem Verfahren gemäß einem der Ansprüche 1 bis 5 erhaltene Fraktion P-2 oder F-2 aufweist.

8

## 0 081 881

12. Synthetisches Schmiermittel oder Dieseltreibstoff-Streckmittel, das als Basis eine nach einem Verfahren gemäß einem der Ansprüche 1 bis 5 erhaltene Fraktion F-2 aufweist.

**Revendications**

1. Procédé de fractionnement au solvant de stéarine d'huile de palme ou de stéarine d'huile de palme transposée catalytiquement pour obtenir, comme première fraction, une graisse glycéridique dure ayant un point de fusion supérieur à 45°C, comme deuxième fraction une fraction glycéridique semi-solide ressemblant à du beurre de cacao par ses propriètés de fusion et ayant une plage de fusion de plus de 33°C à moins de 44°C et comme troisième fraction une huile liquide à la température ambiante, le procédé étant caractérisé en ce qu'il comprend les étapes consistant:

(a) à dissoudre la stéarine dans un solvant, la proportion de solvant étant de 1,0 à 6,0 ml par gramme de stéarine, à chauffer le mélange à environ 50°C pour assurer la dissolution totale;

(b) à faire cristalliser la solution venant de l'étape (a) à 12—14°C;

(c) à recueiller le précipité formé dans l'étape (b) par filtration ou par centrifugation;

(d) à transférer le précipité recueilli de l'étape (c) dans un récipient convenable et à extraire ledit précipité avec du solvant neuf refroidi à 2°C au-dessous de la température de cristallisation et égal en volume à environ deux fois le volume du précipité;

(e) à filtrer la suspension de l'étape (d) sous vide ou sous pression ou par centrifugation et à recueillir le précipité, ce qui donne après traitement dans l'évaporateur et dans l'appareil de désodorisation la première fraction douce (P-1) dépourvue de solvant, ou fraction de graisse dure fondant au-dessus de 45°C.

(f) à cristalliser ensuite le filtrat venant de l'étape (c) à une température de 1 à 3°C;

(g) à recueiller le précipité formé dans l'étape (f) par filtration ou centrifugation;

(h) à transférer le précipité recueilli de l'étape (g) dans un récipient convenable et à extraire ledit précipité avec du solvant neuf refroidi à 0°C et égal en volume à deux fois celui du précipité;

(i) à filtrer la suspension de l'étape (h) par filtration sous vide ou sous pression ou par centrifugation et à recueillir le précipité qui, après traitement dans l'évaporateur et dans l'appareil de désodorisation, donne la deuxième fraction douce (P-2) dépourvue de solvant, qui est une fraction de matière grasse pour confiserie, de point de fusion supérieur à 33°C et inférieur à 44°C;

(j) à traiter le filtrat recueilli de l'étape (g) dans l'évaporateur et l'appareil de désodorisation pour produire la troisième fraction douce (F-2) dépourvue de solvant, à savoir la fraction d'huile.

2. Procédé suivant la revendication 1, dans lequel le solvant est l'acétone.

3. Procédé suivant la revendication 1, dans lequel le solvant est la méthyléthylcétone, la méthyliso-butylcétone, l'alcool méthylique, éthylique ou isopropylique, le 1- ou le 2-nitropropane ou un hydrocarbure mixte à point d'ébullition inférieur à 130°C.

4. Procédé discontinu suivant la revendication 1, 2 ou 3, dans lequel une agitation modérée à 35—70 tr/min est prévue pour accélérer les étapes de cristallisation.

5. Procédé continu suivant la revendication 1, 2, ou 3, dans lequel des durées de séjour modérées de 5 à 50 minutes sont prévues dans des appareils de cristallisation à surface raclée pour accélérer les étapes de cristallisation et pour former les polymorphes cristallins de grosseur correcte.

- 6. Matière grasse pour confiserie, comprenant 1 à 99% en poids de fraction (P-2) obtenue par le procédé suivant l'une quelconque des revendications précédentes et 1 à 99% en poids de beurre de cacao.

7. Matière grasse pour confiserie à point de fusion supérieur à celui du beurre de cacao, comprenant un mélange de 5 à 20% en poids de fraction (P-1) et de 80 à 95% en poids de fraction (P-2) les fractions (P-1) et (P-2) étant préparées par un procédé suivant l'une quelconque des revendications 1 à 5.

8. Huile pour salade, pour la cuisine ou pour la friture, comprenant 1 à 99% en poids de fraction (F-2) obtenue par un procédé suivant l'une quelconque des revendications 1 à 5 et 1 à 99% en poids d'huile d'olive, d'huile, d'arachide, d'huile de soja, d'huile de mais, d'huile de palme, d'huile de graine de tournesol, d'huile de cartame ou d'huile de colza.

9. Graisse végétale ou margarine spéciale comprenant un mélange des fractions (P-1) et (F-2) obtenues par un procédé suivant l'une quelconque des revendications 1 à 5.

10. Produit cosmétique ou pharmaceutique ayant comme base la fraction (P-1) obtenue par un procédé suivant l'une quelconque des revendications 1 à 5.

11. Produit cosmétique ou pharmaceutique ayant comme base la fraction (P-2) ou (F-2) obtenue par un procédé suivant l'une quelconque des revendications 1 à 5.

12. Lubrifiant synthétique ou diluant pour carburant Diesel ayant comme base la fraction (F-2) obtenue par un procédé suivant l'une quelconque des revendications 1 à 5.

DSC Thermograms - Hard Fat Fractions, P-1

Palm Oil Stearine

Rearranged Palm Oil Stearine

Fig.1

TEMP. °C

TEMP. °C

0 081 881

DSC Thermograms - Confectionery Fat Fractions, P-2

Palm Oil Stearine
Confectionery Fat

Rearranged Palm Oil Stearine
Confectionery Fat

TEMP. °C

TEMP. °C

Fig. 2

0 081 881

DSC  Thermogram - Cocoa Butter

Cocoa Butter

TEMP. °C

Fig. 3

0 081 881

DSC Thermograms - Oil Fractions

Palm Oil Stearine

-10    0    10
TEMP. °C

Fig. 4

Rearranged Palm Oil Stearine

-10    0    10    20
TEMP. °C

4

DSC Thermogram - Salad Oil

Fig. 5